# EUROPEAN PATENT SPECIFICATION

(11) **EP 3 909 589 B1**
(45) Date of publication and mention of the grant of the patent: **16.08.2023**
(21) Application number: 21172911.6
(22) Date of filing: 08.05.2021
(51) Int. Cl.: A61K 33/04, A61K 33/06, A61K 33/10, A61K 33/14, A61K 33/16, A61K 33/18, A61K 35/742, A61K 35/745, A61K 35/747, A61K 36/28, A61K 36/47, A61K 36/484, A61P 1/00, A61P 1/04, A61P 43/00, A23L 33/105, A23L 33/135

(54) **LIQUID PROBIOTIC-HERBAL PREPARATION**
FLÜSSIGE PROBIOTISCH-PFLANZLICHE ZUSAMMENSETZUNG
PREPARATION LIQUIDE CONTENANT DES PROBIOTIQUES ET DES PLANTES

(30) Priority: 11.05.2020 PL 43388020; 11.05.2020 EP 20461534
(43) Date of publication of application: 17.11.2021
(73) Proprietor: "Jantar" Wody Mineralne Sp z o.o., 78-100 Kolobrzeg (PL); Kwolek, Dariusz, 00-950 Warszawa (PL)
(72) Inventor: Kwolek, Dariusz, 00-950 Warszawa (PL)
(74) Representative: Grzelak, Anna

(56) References cited:
- WO-A1-2019/157585
- CN-A- 1 048 969
- CN-A- 1 090 983
- CN-A- 104 921 125
- CN-A- 110 604 748
- GHANSHYAM B. DUDHATRA ET AL: "A Comprehensive Review on Pharmacotherapeutics of Herbal Bioenhancers", THE SCIENTIFIC WORLD JOURNAL, vol. 2012, 1 January 2012 (2012-01-01), pages 1-33, XP055438485, DOI: 10.1100/2012/637953

## Description

### TECHNICAL FIELD

The object of the invention is a composition of a functional probiotic-herbal preparation in liquid form comprising herbal extracts from *Phyllanthus emblica, Glycyrrhiza glabra, Stevia rebaudiana* and bacterial strains of *Lactobacillus plantarum* PCM 493, *Bacillus coagulans* DSM 2383, *Lactobacillus reuteri* DSM 17509, *Bifidobacterium long subs. infantis* DSM 20088, *Lactobacillus rhamnosus* DSM 20245, *Lactobacillus casei* DSM 20011 with pro-health and medicinal properties, and uses thereof.

### STATE OF ART

Probiotics are selected strains with documented pro-health properties.

According to FAO/WHO recommendations, only microorganisms isolated from human gastrointestinal tract can be called probiotics. Studies indicate that strains with probiotic characteristics are also isolated from fermented products of animal origin as well as from nondairy fermented products. Many unique collections of microorganisms isolated from traditional and regional fermented foods or from living organisms have been accumulated. Bacterial strains isolated from fermented foods have not only technological usefulness but also probiotic properties, can colonize the human colon and affect its proper functioning.

Traditional fermented foods are abundant sources of microorganisms, some of which exhibit probiotic properties. Such products include food products comprising live lactic fermentation probiotic bacteria belonging to the genera *Lactobacillus* and *Bifidobacterium.* Bacteria in the genus *Lactobacillus* include 117 species, of which the acidophilus, casei, paracasei, and rhamnosus groups are intestine-specific. The genus *Bifidobacterium* comprises 36 species, grouped based on metabolic characteristics and ecological originality. They are microorganisms found mainly in the gastrointestinal tract of humans and animals.

Among the known bacterial species with probiotic properties can be listed:
*Lactobacillus plantarum* - one of the most popular probiotic bacteria, fermenting both dairy products and silages, produces bacteriocins - substances with antibiotic activity, have regenerative effect on intestinal mucosa;
*Bacillus coagulans* - a probiotic strain with highly rated properties, the most resistant to external environmental conditions of all known, strongly adaptogenic in itself, an inhibitor of pathogenic strains in relation to intestinal flora, causes better regeneration of the body after exercise, has a general anti-inflammatory acivity, and analgesic, as a few forms stable spore forms, and thus perfectly colonizes the intestine;
*Lactobacillus reuteri* - a bacterium with a wide range of properties regulating the balance of intestinal bacteria, prevents the proliferation of harmful bacterial flora, supports immunity, colonizes the intestine very well competing with pathological strains, produces reuterin with antibiotic characteristics;
*Bifidus infantis* - is a lactic acid bacterium that synthesizes a particularly high amount of it, has selective biocidal properties against pathogenic strains;
*Lactobacillus rhamnosus -* the most studied group of strains of probiotic bacteria, alone meets the characteristics of an ideal probiotic, shows strong antagonistic properties against pathogenic strains, also produces bacteriocins with antibiotic properties, while producing lactic acid, regulates the pH in the intestine;
*Lactobacillus casei* - bacilli of this strain carry out fermentation of glucose and produce lactic acid, it is a well-known lactic and silage bacterium.

The biodiversity of probiotics results primarily from the fact that each microorganism is characterized by specific properties and range of action. This allows both solving specific human and animal health problems. Usually, however, antimicrobial properties are of primary importance, hence this characteristic is among the main selection criteria of most known probiotics. The mechanism of action of probiotics is based on their inhibition of pathogenic microflora in the human gastrointestinal tract by supplying competing microorganisms with beneficial effects. This beneficial effect may be related to the adhesion of probiotic bacteria to the intestinal mucosa, which reduces the possibility of adhesion of other organisms, and may be based on growth antagonism.

Among the characteristics of an optimal probiotic there are resistance to gastric acids and bile, adherence to human intestinal cells, ability to colonize it and to produce antibacterial substances, rapid growth rate and safety of use.

In recent years, the field of human nutrition science has seen a strong development in the use of probiotics and the determination of their effects on human health, also due to rising costs of medical treatment and the desire to extend and improve the quality of life, there is a growing public interest in products with additional nutritional properties and biological properties.

The global probiotic products market was estimated at US$ 24 x 109 in 2018. More than 500 new probiotic products from the food and beverages segment (Food and Beverages) have been introduced into the market in the last decade. These products have been accepted by consumers to varying degrees, but mainly due to their overall pro-health properties. For example, probiotic original chocolates held a larger market share in 2014 than probiotic cheese and butter because these products have more pro-health properties. Equally important factors for larger market share are taste and convenience of use.

Consumers, especially children, the elderly, and people with active lifestyles, do not always willingly reach for preparations in the form of tablets or powders that are difficult to dissolve, but are more likely to consume ready-to-drink liquid preparations. All age groups and cultures enjoy consuming water or water-based beverages. In many cultures, mineral waters with specific pro-health properties, including waters supplementing macro- and microelement deficiencies, are known to be willingly drunk by consumers as well as used for pro-health purposes.

Mineral waters are known to be used in crenotherapy. The beneficial properties of alkaline waters are used in the treatment of diseases of the digestive system, especially the stomach and during intensive exercise, when due to lactic acid secretion in muscles, acidification of the body occurs. Chloride and sodium waters are used to ensure internal water-electrolyte balance. Waters containing iodine and selenium support functioning of the thyroid gland and positively affect brain function. Waters comprising bicarbonate, HCO₃, improve the assimilability of calcium and magnesium. Medium / average mineralized spring water extracted from the Permian deposit in Kolobrzeg is known, for example, the mineral water Jantar (Jantar Wody Mineralne, Poland) with a slightly alkaline pH of about 7.4, thereby counteracting the acidification of the body, which contains chloride, bicarbonate, sodium, calcium, magnesium, potassium, fluorine, iodine, selenium and many other valuable mineral ingredients.

Most probiotic bacteria require a slightly acidic environment to maintain their properties and ensure long-term storage, and their stable storage and preservation in even slightly alkaline waters is difficult.

The intestinal microbiota has an effect on all cells of the body. Proper probiotic supplementation supplements and compensates for civilization's probiotic deficiencies. However, the greatest pro-health effect is seen in the so-called probiotic immunology - this is the extent of modulation of major signaling pathways and secreted cytokines by the immune system, along with the lymphatic system, i.e. the entire complex of activities having their basis in a properly composed intestinal bacterial flora (GALT, MLN, FAE systems, transcription factors NFkB, MAPK, TLR, etc.).

Liquid probiotics are known, with a very small product share compared to capsule probiotics.

Herbal extracts with adaptogenic action are known in the state of the art. Such action is demonstrated, for example, by extracts from:
*Phyllanthus embolic* - also known as Indian gooseberry, is an herb originating from India, used to improve digestion, lowers blood sugar levels, lowers cholesterol levels, has a cough-reducing effect, tones the nervous system, and supports regeneration after physical and mental exertion;
*Glycyrrhiza gabra* - a plant known in Europe as licorice, the use of which helps with stomach ulcers, is a good expectorant, supports liver function, has a strong activating effect on neurotransmission, supports the body's immunity;
*Stevia rebaudiana* - otherwise honey leaf or stevia, a plant from which a sweetener is produced to replace sugar, lowers blood sugar levels, regulates metabolism, regulates the acid-base balance of the body, lowers high blood pressure, has antibacterial, anti-inflammatory and immunostimulating properties.

CN1090983A discloses a nutritious health-care drink containing natural mineral matter. It is made up by blending natural mineral water as solvent with tuber type nurient food, *Stevia rebaudiana,* white sugar and dry ice as solutes.

WO2019157585A1 discloses probiotic formulations, products of manufacture and kits comprising them, and methods of using them, for the treatment and alleviation of metabolic and oxidative stress, inflammation and neurodegeneration. The probiotic formulations comprise or consist any combination of the three probiotic strains: *Lactobacillus fermentum* NCIMB 5221, *Lactobacillus plantarum* NCIMB 8826 and *Bifidobacteria longum* spp. *infantis* NCIMB 702255. The probiotic formulation according to WO2019157585A1 are used for: the treatment of metabolic conditions such as diabetes and obesity, inflammatory conditions such as IBD, IBS, arthritis; and, neurodegenerative diseases such as Alzheimer's disease and Parkinson's disease, and arealso used for: resisting oxidative stress, reducing inflammation, lowering blood and total glucose levels, lowering triglycerides levels and managing insulin resistance.

The literature describes many probiotic strains and their properties, but usually individually.

There is no view on the complementary, mutually reinforcing, synergistic effect of a greater number of bacterial strains, as a total combined therapy, also liquid preparations containing probiotic consortia of bacteria interacting with plant extracts with pro-health properties are not known.

### SUMMARY OF INVENTION

In order to meet these trends and overcome disadvantages indicated in the state of the art, the aim of the invention was to develop a liquid probiotic pro-health composition in the form of a functional beverage composition - a liquid dietary supplement comprising probiotic bacterial strains and herbal extracts exhibiting pro-health activity through the obtained effect of their synergistic interaction, strengthened by the use, as the main component, of spring water of a particular type and of a particular composition, i.e. the water from the Permian deposit in Kolobrzeg, interacting through its composition of micro- and macroelements in obtaining and strengthening the final pro-health effect on the human body of particular ingredients of the composition of the functional beverage according to the invention.

Unexpectedly, it turned out that it is possible to produce a stable functional beverage based on a probiotic-herbal preparation, based on slightly alkaline, medium mineralized mineral water from the Permian deposit in Kolobrzeg, in the form of a complex consortium of probiotic strains and a mixture of properly selected herbal extracts, as a probiotic intended for prevention or supportive treatment of many disease entities, as well as intended for daily consumption for the group oriented at healthy lifestyle, improvement of health and fitness, and prevention of civilization diseases.

Due to its selected qualitative and quantitative constitution this composition is tasty, does not require sweetening (can be used by diabetics and people on a diet), the bacterial strains suspended in it are stable and is suitable for long storage despite the lack of the use of preservatives.

The probiotic bacteria included in the composition not only have a positive effect on health, enhancing probiotic immunology, but are also characterized by a number of properties that will distinguish them as safe, functional and suitable for industrial use, which properties are used to achieve the proper composition and stability of the functional beverage composition according to the invention and uses thereof.

The object of the invention relates to a composition of a functional probiotic-herbal preparation in liquid form, which comprises
- 70-90 % vol. of medium mineralized mineral water comprising chloride, bicarbonate, sodium, calcium, magnesium, potassium, fluoride, iodine, selenium, with a slightly alkaline pH;
- 3.2 - 13.7 % vol. liquid herbal extract from *Phyllanthus emblica* - water, 35% alcohol, Drug Extract Ratio (DER): 1:15;
- 1.7 - 6 % vol. liquid herbal extract from *Glycyrrhiza glabra* - water, 32 % alcohol, DER: 1:25;
- 1.5 - 6 % vol. *Stevia rebaudiana* liquid herbal extract - water, DER: 1:25;
- 0.7 % vol. of *Lactobacillus plantarum* PCM 493 strain (Polish Collection of Microorganisms) with a minimum content of 10 _{*} 10⁹ CFU/ml;
- 0.7 % vol. of *Bacillus coagulans* DSM 2383 strain (DSMZ - German Collection of Microorganism and Cells Cultures GmbH) with a minimum content of 2 _{*} 10⁹ CFU/ml;
- 0.5 % vol. of *Lactobacillus reuteri* DSM 17509 strain (DSMZ - German Collection of Microorganism and Cells Cultures GmbH) with a minimum content of 10 _{*} 10⁹ CFU/ml;
- 0.7 % vol. of *Bifidobacterium longum subs. infantis* DSM 20088 strain (DSMZ - German Collection of Microorganism and Cells Cultures GmbH) with a minimum content of 5 _{*} 10⁹ CFU/ml;
- 1 % vol. of *Lactobacillus rhamnosus* DSM 20245 strain (DSMZ - German Collection of Microorganism and Cells Cultures GmbH) with a minimum content of 10 _{*} 10⁹ CFU/ml;
- 0.7 % vol. of *Lactobacillus casei* DSM 20011 strain (DSMZ - German Collection of Microorganism and Cells Cultures GmbH) with a minimum content of 10 _{**} 10⁹ CFU/ml.

In the preferred composition of the functional probiotic-herbal preparation in liquid form, the medium mineralized mineral water is spring water extracted from the Permian deposit in Kolobrzeg, more preferably Jantar mineral water.

Preferably, the composition of a functional probiotic-herbal preparation in liquid form is of pH 7.4The invention also relates to a composition of a functional probiotic-herbal preparation in liquid form according to the invention for use as a medicament.

The invention also relates to a composition of a functional probiotic-herbal preparation in liquid form according to the invention for use as a medicament or adjunct in the treatment, alleviation and/or prevention of entities and disease conditions selected from:
digestive disorders, diarrhea, obesity, cardiovascular disease, gastric or duodenal ulcer diseases, fatty liver and its consequences, hepatic encephalopathy, intestinal disorders of various origins, including specific and non-specific inflammatory conditions and of autoimmune origin, type II diabetes, metabolic disorders and disease entities resulting from them, allergies, hormonal disorders, chronic fatigue syndrome, occupational burnout syndrome, depression and low mood states, digestive system disorders resulting from antibiotic therapy.

Preferably, the composition for use as a medicament or adjunct is used to treat, alleviate and/or prevent the onset of inflammatory intestinal disorders, irritable bowel syndrome, occupational burnout and/or low mood states.

The invention also relates to a composition of a functional probiotic-herbal preparation in liquid form according to the invention for use as a liquid probiotic agent.

The invention also relates to a composition of a functional probiotic-herbal preparation in liquid form according to the invention for use as a liquid symbiotic supplement.

The invention also relates to the non-medical use of a composition of a functional probiotic-herbal preparation in liquid form according to the invention as a symbiotic, probiotic, dietary supplement and/or functional beverage for supplementing, stabilizing the microbiome and/or restoring the proper gastrointestinal microbiome in an individual.

The invention relates to an improved probiotic in liquid form having the characteristics often postulated in the literature as an ideal probiotic that exhibits:
- the ability to colonize the gastrointestinal tract,
- the individual microorganisms included in its composition originate from the human intestinal biome,
- exhibits antagonism towards pathogenic bacteria of the human digestive system,
- the microorganisms in its composition exhibit the ability to adhere to the intestinal epithelium,
- the microorganisms in its composition produce antimicrobial substances against bacteria undesirable in the human intestinal biome,
- exhibits resistance to the gastrointestinal tract environment (ability to survive in the presence of gastric acid, bile and pancreatic enzymes),
- exhibits viability and activity during intestinal passage,
- exhibits viability and stability during storage,
- exhibits safety of use through lack of adverse effects,
- exhibits clinically documented pro-health effects on the human body.

The invention was developed on the basis of analyses of the human intestinal biome (microbiome), as a diverse system described in the literature, and on the basis of long-term studies and observations of patients, who were prescribed the use of individual probiotics individually and in various combinations, as well as in combination with selected herbal extracts in deliberately planned combination therapies.

In the final selection of probiotic strains included in the composition according to the invention, the properties of known pathogenic bacterial strains and the conditions caused by them were taken into account, as well as the effect of probiotic intestinal microflora on intestinal functions with demonstration of its mechanism. Particular attention was paid to the ability of bacterial strains to synthesize specific substances in the human intestine, specifically neurotransmitters and hormones affecting the gut-brain axis. Thus, using the composition of a functional beverage according to the invention, the effect of strengthening probiotic immunology was achieved and significant pro-health effects were obtained in people taking such a composition.

The results of many years of observation of patients on given combinations of preparations using various types of non-mineral and mineral waters as a substrate for administration of combinations of probiotic organisms and plant extracts led to the development of a composition of a complex herbal-bacterial preparation in liquid form based on mineral water from the Permian deposit in Kolobrzeg (Jantar intake), being the subject of the present application.

### DETAILED DESCRIPTION

The probiotic strains comprised in the functional beverage composition according to the invention have pro-health and therapeutic benefits demonstrated in the literature and are approved for marketing as probiotics for use in humans.

Selection of the product composition resulted from research and development work based on the analysis of registered bacterial strains with probiotic indications in terms of their pro-health and therapeutic properties and such selection of their combination, water base and herbal extracts to obtain a visibly greater effect from the combined interaction than from the effect observed for a single action of each of these factors, the effect of self-conserving and self-stabilizing beverage, not requiring sweetening while providing a pleasant taste experience.

Unexpectedly, it turned out that the combination of appropriately selected probiotic strains with herbal extracts with demonstrated adaptogenic activity provides a targeted biochemical-metabolic action: of both bacterial strains and herbal active substances - towards the formation of neurotransmitters and thus having a combined effect on the gut-brain axis. This is reflected in the clinically validated spectrum of applications, with respect to the disease entities or therapeutic areas further listed in the application.

The mechanism of action of the composition of a functional probiotic-herbal preparation in liquid form according to the invention in a holistic and synergistic approach provides:
a) Stabilization of the intestinal barrier by, among other things: improving the tightness of the intestinal epithelium, increasing the number and strengthening the structures of intercellular protein bonds, thickening the mucus layer and improving its density, providing enhanced bacterial adhesion to the inner wall of the intestinal mucosa and creating a protective "film", strengthening the integrity of the lipid barrier.
b) It affects immunomodulation by, among other things: maintaining GALT homeostasis, stimulating immune response (especially IgA response) and production of anti-inflammatory cytokines (e.g. IL-10), increasing phagocytosis capacity (granulocytes and macrophages), activating NK cells, preventing apoptosis, enhancing specific antibody response to pathogenic organisms.
c) Signaling in the brain-gut axis - through effects on mechanisms: neuronal (ENS), neuroendocrine (HPA), neuroimmune and the ability to synthesize various neurotransmitters and neuromodulators.
d) Signaling and internal secretion in the axis: gut - brain - liver, thus affecting the manifestation of systemic symptoms of hepatic etiology, including neurological and psychiatric symptoms.

By administering to individuals with various disorders and various disease states the composition of a functional probiotic-herbal preparation in liquid form according to the invention, its preventive, alleviating, curative and/or pro-health effects on disease entities and conditions selected from: digestive disorders, diarrhea, obesity, cardiovascular diseases, gastric and duodenal ulcer diseases, fatty liver and its consequences (alcoholic and non-alcoholic), hepatic encephalopathy, intestinal disorders of various origins, including specific and non-specific inflammatory conditions and of autoimmune origin, type II diabetes, metabolic disorders and disease entities resulting from them (etiological), allergies, hormonal disorders, chronic fatigue syndrome, occupational burnout, depression and low mood states.

In the prepared composition of the functional probiotic-herbal preparation in liquid form according to the invention, solution stabilization in relation to the solvent, i.e. pH of the spring water extracted from the Permian deposit in Kolobrzeg, of Jantar type, of pH about 7.4 has been achieved.

Whereas the pH of the obtained composition is slightly changed in relation to the initial one and amounts for the final solution, i.e. with probiotics and herbal extracts, to pH about 7.8.

Unexpectedly, it turned out that application of appropriately selected extracts of herbal mixture (extracts from *Phyllanthus emblica, Glycyrrhiza glabra, Stevia rebaudiana*) brings not only pro-health effects, because by using the mixture of herbal extracts in the whole formulation of the liquid, increased stability and stability of the product was achieved - unlike in the case of probiotics dissolved in the liquid, which base for dissolution was only spring water extracted from Permian deposit in Kolobrzeg of Jantar type. The presence of a conglomerate of active substances, used in the herbal mixture used in the composition, stabilizes the probiotic solution in Jantar type water and prevents the processes that would reduce its usefulness for consumption. In a solution of such a composition as in the composition of a functional probiotic-herbal preparation in liquid form according to the invention, no decomposition reactions or causing spoilage or instability of the beverage occur, and the probiotics obtain long survival and shelf life. Without being bound by any theory - deriving this from the biochemistry of transformations - it appears that this effect is due to the presence of polysaccharides, perhaps also other compounds, comprised in the mixture of combined plant extracts.

In arriving at the final composition of a functional probiotic-herbal preparation in liquid form according to the invention, a solvent rich in mineral composition was sought which constitutes essential minerals being catalysts for the work of digestive enzymes in the human digestive system. It was also sought for a solvent of suitable pH having such minerals in its composition. The most similar to this assumption and fulfilling its function as a pro-health "catalyst" for the selected consortium and plant extracts turned out to be the spring water extracted from the Permian deposit in Kolobrzeg.

The invention ultimately constitutes such a Jantar type water based mixture, which, in the action of intestinal biochemistry and enzymatic metabolism, supports in a sustainable manner the colonization of the bacterial strains present in the probiotic, allowing their colonization in the intestine in a reliable and complete manner. This is aided by the polysaccharides and glycosides present in the herbal extracts, acting as a prebiotic. As a result, the probiotic beverage produced according to the invention functions according to the classification of dietary supplements, as a symbiotic, in the registration category: dietary supplement for special nutritional use. The mineral composition derived from Jantar water and herbs constitutes and ensures adequate buffering of the stomach content, so that the bacterial consortia comprised in the composition of the functional probiotic-herbal preparation in liquid form according to the invention, during their transit through the gastrointestinal tract to the place of colonization, are not digested but pass in a large proportion, desirable and sufficient, to the large intestine, in order to colonize it.

Additionally, this mineral composition ensures better adherence to the intestinal mucosa in the biochemical passage and supports the production of a specific protective biofilm on the surface of the intestinal mucosa (influences the so-called mucosal mechanisms).

The use of the composition of functional probiotic-herbal preparation in liquid form according to the invention instead of the composition in dry form causes lack of necessity to use many additional substances originating from chemical synthesis such as: alginates, gums, carrageenan, modified milk proteins, ascorbic acid, monosodium glutamate, maltodextrins, gelatin, starch, which are necessary and indispensable in the most popular and commonly used capsule formulations.

Additionally, when producing the composition of functional probiotic-herbal preparation in liquid form according to the invention we avoid (in comparison with capsule with lyophilized bacteria) losses on bacterial consortia resulting from thermal treatment (in biomass lyophilization) or bacterial cell damage (in spray drying). Moreover, the capsule form requires appropriate temperature and humidity to preserve the viability of the bacteria during transport and storage, which is not important in the developed liquid form that ensures stabilization. Therefore, the liquid form simplifies the technological process of production of the formulation according to the invention and lowers its costs.

In addition, administration of probiotics in liquid form causes that, unlike in the case of dry preparations, also postbiotics are administered - produced by bacteria, which are the products of their metabolism, which increases the adhesive properties of administered probiotics, making them to adhere to the intestinal epithelial cells more effectively than in the case of probiotics administered in capsules.

The adaptogenic action of certain plant extracts on the human body is known. However, there is no known set of herbal extracts with adaptogenic activity, which can be used as an additive to mineral water to produce a composition of a functional probiotic-herbal preparation in liquid form according to the invention, providing an attractive sweet taste without increasing the calorific value of the beverage, at the same time ensuring its stability as well as providing a wide spectrum of adaptogenic action for humans, and thus a pro-health effect.

In order to obtain an acceptable, attractive for consumers taste of the composition of functional probiotic-herbal preparation in liquid form according to the invention and not introducing any sugars, which could be a substrate for fermentation process, proper taste qualities were obtained by selection of quantitative and qualitative composition of herbal extracts and by using the extracts obtained in the extraction process conducted in such a way that they did not lose their taste qualities during their preparation, important for the taste perceived by customers, recipients of the product of functional probiotic-herbal preparation in liquid form according to the invention.

The use of Stevia rebudiana (stevia) in the invention results from its properties in solution, i.e. stevia provides pH stabilization in a wide range (from 2 to 9), does not ferment, exhibits light resistance, has properties of a natural preservative, exhibits very good solubility in water.

In the state of the art, prebiotic-containing dietary supplements with stevia-containing solutions were not known. Thus, stevia extract plays a decisive role as a stabilizer of the entire complex composition.

By using a mixture of extracts from *Phyllanthus emblica* (also known as *Emblica officinalis*), *Glycyrrhiza glabra, Stevia rebaudiana,* the particularly important sweetening ingredient used here being stevia, a sweet taste was achieved, described by the testers as specifically and very pleasantly balanced sweet, which results from the combination of the respective glycosides of these different herbal ingredients. The sweet taste is produced by all herbal ingredients used, whereby the *Stevia rebaudiana* extract is the most important. The addition of Phyllanthus emblica extracts is responsible for the special taste sensation. This is a combination not previously used in dietary supplements and, in the case of the invention, is novel. At the same time, the use of stevia to obtain a sweet taste does not provide any calories, because stevia glycosides are not digested by the human body.

### DESCRIPTION OF EMBODIMENTS

The following examples are included only to illustrate the invention and to explain particular aspects of the invention, not to limit it, and should not be equated with the entire scope of the invention as defined in the appended claims. In the following examples, unless otherwise indicated, standard materials and methods used in the field have been used or manufacturers' recommendations for specific materials and methods have been followed.

### EXAMPLES

The analysis and selection of bacterial strains and composition of herbal extracts used to create a composition of functional beverage according to the invention has been done on the basis of above described criteria, starting from probiotics shielding effect in the case of antibiotic therapy to the demonstration of preventive and curative properties in cases related to the digestive system, taking into account the latest reports on the impact of probiotics on the human intestinal biome conditioning the interaction of the gut-brain-hepatic axis, it being in turn a factor in the etiology of many civilization diseases. As a result of this part of the work, a suitable, universal composition of bacterial strains in combination with selected herbal extracts has been determined for the liquid based on Jantar spring water.

After work on the composition of the mixture, laboratory tests were conducted on selected strains to adapt them to the formulation of probiotic liquid based on Jantar water. Laboratory research related to:
- survival and activity of the bacterial cultures forming the strain mixture formula over time,
- stability and durability of the lyophilized mixture as a whole.

Tested strains meet safety, functionality and technological suitability criteria.

The study was conducted in the options - single lyophilized and dissolved in Jantar water.

After obtaining confirmation of satisfactory parameters of survival, stability and durability, the final approval of the composition was made and, on the basis of the conducted tests, the proportions of the lyophilizate of particular strains and of the whole mixture for a given volume of spring water were established for obtaining pro-health properties.

On the basis of the experiments conducted on research groups, the exact dosage of the composition of functional probiotic-herbal preparation on the basis of mineral water was calculated to be about 1 liter/day/per person, with the division of sipping the beverage throughout the day in arbitrary amounts. In this way, the action of the active substances of the complex composition according to the invention will be maintained in a permanent cycle.

The final product in the form of a composition reflects the latest research results and meets the demand for supportive action in the prevention of civilization diseases by supplementing the normal composition of the human intestinal biome as a dietary supplement or functional beverage.

In the examples given below, a probiotic dosage comprising, in each dose, 5ml of probiotic (having a final CFU count corresponding to the amount of a given strain indicated in the range given in Example 5.B per ½ liter of the final liquid composition according to the invention, equivalent to the dose of the given probiotic in ½ liter of the composition according to the invention) and 300 mg of dry lyophilized herb extract (having a final amount in mg of liquid extract corresponding to the amount of extract of the given species indicated in the range given in Example 5.B per ½ liter of the final liquid composition according to the invention, equivalent to the dose of the respective adaptogen in ½ liter of the composition according to the invention) of each of the adaptogens recommended in the example and at a frequency of 2 times per day for both groups of substances was recommended.

### Example 1

Three different study preparations were administered to 7 patients from the Medical Center in Warsaw with a diagnosis of inflammatory bowel disorders, who were divided into 3 study groups, with the control group consisting of individuals with the same disorder who received standard pharmacological treatment. Individuals in a specific research group were administered respectively:
- group 1.1- single probiotics in 2 people *(Lactobacillus rhamnosus* DSM 20245);
- group 1.2 - double probiotics in 2 people *(Lactobacillus rhamnosus* DSM 20245 + *Lactobacillus* casei DSM 20011),
- group 1.3 - 3 people received 4 total probiotics (*Bacillus coagulans* DSM 2383 + *Lactobacillus plantarum* PCM 493 + Lactobacillus *casei* DSM 20011 + *Lactobacillus rhamnosus* DSM 20245).

Obtained observations of such supplementation resulted in conclusions that the effect of cessation of complaints or their alleviation appeared in group 1.3 with a wider spectrum of probiotic supplementation, moreover, one person with additional depressive disorders from this group 1.3 reported a significant improvement in mood.

### Example 2

In a group of 8 people with symptoms of occupational burnout, were administered:
5 individuals probiotics in a composite of the 4 strains listed in the application (*Bifidobacterium long subs. infantis* DSM 20088 + *Lactobacillus reuteri* DSM 17509 + *Lactobacillus plantarum* PCM 493 + Bacillus *coagulans* DSM 2383), and
a group of 3 individuals were prescribed only the adaptogens ( a mixture of lyophilized herbs from *Phyllanthus emblica, Glycyrrhiza glabra,* and *Stevia rebaudiana*) listed in the application. The control group consisted of individuals with the same disorder who received no drug treatment.

In both study groups, relief of symptoms was observed.

### Example 3

A group of 12 patients on three supplementation programs with a diagnosis of irritable bowel syndrome (IBS) were analyzed. Individuals in a specific study group were administered respectively:
- group 3.1 - 4 people single probiotic *(Lactobacillus rhamnosus* DSM 20245),
- group 3.2 - 3 people compound probiotic (*Bacillus coagulans* DSM 2383 + *Lactobacillus plantarum* PCM 493 + Lactobacillus *casei* DSM 20011 + *Lactobacillus rhamnosus* DSM 20245),
- group 3.3 - 5 people single probiotic *(Lactobacillus rhamnosus* DSM 20245) together with adaptogens (a mixture of lyophilized herbs from *Phyllanthus emblica, Glycyrrhiza glabra* and *Stevia rebaudiana).*

Among patients supplemented with a single probiotic in group 3.1. two individuals reported no improvement and one of them confirmed slight alleviation of symptoms, in group 3.2. receiving a compound probiotic the patients communicated medium alleviation of symptoms, in group 3.3. i.e. individuals receiving a combination therapy of probiotic + adaptogens all patients communicated significant improvement.

### Example 4

Patients in the number of 6, patients of the Medical Center in Warsaw with chronic fatigue syndrome after unsuccessful attempts to treat this condition in various outpatient clinics confirmed positive effects of combined treatment with compound probiotics (*Lactobacillus reuteri* DSM 17509 + *Bifidobacterium long subs. infantis* DSM 20088 + Lactobacillus *rhamnosus* DSM 20245 administered together with adaptogen lyophilizate from *Glycyrrhiza glabra* after administration of the mixtures for two consecutive weeks.

### Example 5

### Preparation of a composition of functional probiotic-herbal preparation in the liquid form based on Jantar spring water

### 5.A. Preparation of a functional probiotic-herbal preparation in liquid form administered to probands and individuals with various disease states

For the preparation of 1 liter was used:
- 700 ml - Jantar mineral spring water from the Permian deposit in Kolobrzeg (manufacturer: Jantar Wody Mineralne, Poland);
- 137 ml - liquid herbal extract *Phyllanthus emblica* (*Emblica officinalis*) - water, 35 % alcohol, DER: 1:15 (manufacturer: Kaczmarek - Komponenty, Mrowino);
- 60 ml - liquid herbal extract *Glycyrrhiza glabra* - water, 32 % alcohol, DER: 1:25 (manufacturer: Melissa, Brzeziny);
- 60 ml - liquid herbal extract *Stevia rebaudiana* - water, DER: 1:25 (manufacturer: Greenvit, Zambrow);
- 7 ml of *Lactobacillus plantarum* PCM 493 (purchased from Polish Collection of Microorganisms) with a minimum content of 10 _{*} 10⁹CFU/ml;
- 7 ml of *Bacillus coagulans* DSM 2383 (purchased from DSMZ - German Collection of Microorganism and Cells Cultures GmbH) with a minimum content of 2 _{*} 10⁹ CFU/ml;
- 5 ml of *Lactobacillus reuteri* DSM 17509 (purchased from DSMZ - German Collection of Microorganism and Cells Cultures GmbH) with a minimum content of 10 _{*} 10⁹ CFU/ml;
- 7 ml of *Bifidobacterium long subs. infantis* DSM 20088 (purchased from DSMZ - German Collection of Microorganism and Cells Cultures GmbH) with a minimum content of 5 _{*} 10⁹ CFU/ml;
- 10 ml of *Lactobacillus rhamnosus* DSM 20245 (purchased from DSMZ - German Collection of Microorganism and Cells Cultures GmbH) with a minimum content of 10 _{*} 10⁹ CFU/ml;
- 7 ml of *Lactobacillus casei* DSM 20011 (purchased from DSMZ - German Collection of Microorganism and Cells Cultures GmbH) with a minimum content of 10 _{*} 10⁹ CFU/ml.

**5.B.** The biological functionality and assumed biological properties were observed and confirmed on individual probands and treated individuals for formulation mixtures for which the following ingredients were used to create in the following volume proportions:
- Jantar mineral spring water from the Permian deposit in Kolobrzeg (manufacturer: Jantar Wody Mineralne, Poland ) 70 - 90 % vol.;
- liquid herbal extract *Phyllanthus emblica (Emblica officinalis)* - water, 35 % alcohol, DER: 1:15 (manufacturer: Kaczmarek - Komponenty, Mrowino) 3.2 - 13.7 % vol.;
- liquid herbal extract *Glycyrrhiza glabra* - water, 32 % alcohol, DER: 1:25 (manufacturer: Melissa, Brzeziny) 1.7 - 6 % vol.;
- liquid herbal extract *Stevia rebaudiana* - liquid herbal extract, water, DER: 1:25 (manufacturer: Greenvit, Zambrow) 1.5 - 6 % vol.;
- liquid solution comprising *Lactobacillus plantarum* PCM 493 strain (purchased from Polish Collection of Microorganisms) with a minimum content of 10 _{*} 10⁹ CFU/ml - 0.7 % vol.;
- liquid solution comprising *Bacillus coagulans* DSM 2383 strain (purchased from DSMZ - German Collection of Microorganism and Cells Cultures GmbH) with a minimum content of 2 _{*} 10⁹ CFU/ml - 0.7 % vol.;
- liquid solution comprising *Lactobacillus reuteri* DSM 17509 strain (purchased from DSMZ - German Collection of Microorganism and Cells Cultures GmbH) with a minimum content of 10 _{*} 10⁹ CFU/ml - 0.5 % vol.;
- liquid solution comprising *Bifidobacterium long subs. infantis* DSM 20088 strain (purchased from DSMZ - German Collection of Microorganism and Cells Cultures GmbH) with a minimum content of 5 _{*} 10⁹ CFU/ml - 0.7 % vol.;
- liquid solution comprising *Lactobacillus rhamnosus* DSM 20245 strain (purchased from DSMZ - German Collection of Microorganism and Cells Cultures GmbH) with a minimum content of 10 _{*} 10⁹ CFU/ml - 1 % vol.;
- liquid solution comprising *Lactobacillus casei* DSM 20011 strain (purchased from DSMZ - German Collection of Microorganism and Cells Cultures GmbH) with a minimum content of 10 _{*} 10⁹ CFU/ml - 0.7 % vol.

In the above-mentioned ranges of concentrations of ingredients of the composition according to the invention, a pro-health synergism of plant ingredients providing adaptogenic and prebiotic factors, probiotic bacteria providing also post-biotic ingredients and also exhibiting adaptogenic effects and macro- and microelement ingredients comprised in a particular type of water used for creating the composition according to the invention, which water provides catalysts for enzyme reactions in the human digestive system, were demonstrated.

### LITERATURE

Albesharat R., Ehrmann M. A., Korakli M., Yazaji S., Vogel R. F., 2011. Phenotypic and genotypic analyses of lactic acid bacteria in local fermented food, breast milk and faeces of mothers and their babies. Systematic and Applied Microbiology, nr 34
Ammor S., Tauveron G., Dufour E., Chevallier I., 2006. Antibacterial activity of lactic acid bacteria against spoilage and pathogenic bacteria isolated from the same meat small scale facility 1-Screening and characterization of the antibacterial compounds. Food Control, nr 17 Babuchowski A., Wzorek W., 2003. Technologie fermentacyjne, w Bednarski W., Reps A..
Biotechnologia zywnosci. Wydawnictwo Naukowo-Techniczne
Czarnecki Z., Czarnecka M., 2006. Tradycyjne wykorzystanie mikroorganizmow w produkcji zywnosci, w Gawecki J., Libudzisz Z.. Mikroorganizmy w zywnosci i zywieniu, Wydawnictwo Akademii Rolniczej im. A. Cieszkowskiego w Poznaniu
De Vriesa M. C., Vaughanb E. E., Kleerebezema M.,. de Vos W. M., 2006. Lactobacillus plantarum-survival, functional and potential probiotic properties in the human intestinal tract. International Dairy Journal, nr 16
De Vuyst L., Degeest B., 1999. Heteropolysaccharides from lactic acid bacteria. FEMS Microbiology Reviews, nr 23
De Vuyst L., Vandamme E.J., 1994. Antimicrobial potential of lactic acid bacteria. In: De Vuyst L., Vandamme E.J. (Eds.), Bacteriocins of Lactic Acid Bacteria: Microbiology, Genetics and Applications. Blackie Academic and Professional, London
Dzierzanowska D., 2009. Mikroflora fizjologiczna czlowieka. Opieka paliatywna nad dziecmi, tom 17
Fiedurek J., 2014. Mikrobiom a zdrowie czlowieka. Wydawnictwo Uniwersytetu Marii Curie-Sklodowskiej w Lublinie
Gajewska J., Blaszczyk M. K., 2012. Probiotyczne bakterie fermentacji mlekowej (LAB). Postepy Mikrobiologii, tom 51, nr 1
Goldfinger-Kunicki W. H., Zycie bakterii, Wydawnictwo Naukowe PWN, Warszawa 1998
Gorecki R. K., Bardowski R. K., 2011. Molekularne mechanizmy opornosci bakterii kwasu mlekowego na bakteriofagi. Postepy Mikrobiologii, tom 50, nr 4
Gorska S., Grycko P., Rybka J., Gamian A., 2007. Egzopolisacharydy bakterii kwasu mlekowego - biosynteza i struktura. Postepy Higieny i Medycyny Doswiadczalnej, nr 61
Gorska S., Jarzab A., Gamian A., 2009. Bakterie probiotyczne w przewodzie pokarmowym czlowieka jako czynnik stymulujacy uklad odpornosciowy. Postepy Higieny i Medycyny Doswiadczalnej, nr 63
Gu R. X., Yang Z. Q., Li Z. H., Chen S. L., Luo Z. L., 2008. Probiotic properties of lactic acid bacteria isolated from stool samples of longevous people in regions of Hotan, Xinjiang and Bama, Guangxi, China. Anaerobe, nr 14
Gwiazdowska D., Trojanowska K., 2005. Bakteriocyny - wlasciwosci i aktywnosc przeciwdrobnoustrojowa. Biotechnologia, tom 68, nr 1
Heczko P. B., Strus M., Jawien M., Szymanski H., 2005. Medyczne zastosowanie probiotykow, Wiadomosci Lekarskie, nr 58
Jach M., Los R., Maj M., Malm A., 2013. Probiotyki - aspekty funkcjonalne i technologiczne. Postepy Mikrobiologii, tom 52, nr 2
Jurkowski M., Blaszczyk M., 2012. Charakterystyka fizjologiczno-biochemiczna bakterii fermentacji mlekowej. Kosmos. Problemy nauk biologicznych, tom 61, nr 3
Kraszewska J., Wzorek W., Sztando E., Raczynska-Cabaj A., 2005. Aktywnosc antagonistyczna bakterii fermentacji mlekowej z gatunku Lactobacillus plantarum. Acta Scientiarum Polonorum, tom 1, nr 4
Libudzisz Z., 2004. Mikroflora jelitowa czlowieka a probiotyki. Zakazenia, nr 6
Monteagudo-Mera A., Rodriguez-Aparicio L., Rúa J., Martinez-Blanco H., Navasa N., Garcia-Armesto M. R., Ferrero M. A., 2012. In vitro evaluation of physiological probiotic properties of different lactic acid bacteria strains of dairy and human origin. Journal of Funcional Foods, nr 4 Moraes P. M., Perin L. M., Ortolani M. B. T., Yamazi A. K., Viçosa G. N., Nero L. A., 2010. Protocols for the isolation and detection of lactic acid bacteria with bacteriocinogenic potential. LWT - Food Science and Technology, tom 43, nr 9
Nowak A., Slizewska K., Libudzisz Z., 2010. Probiotyki - historia i mechanizmy dzialania. Zywnosc. Nauka. Technologia. Jakosc., tom 71, nr 17
Olszewska M., Laniewska-Trokenheim L., 2013. Odpowiedz bakterii fermentacji mlekowej na stres - stadium VBNC. Zywnosc. Nauka. Technologia. Jakosc, tom 90, nr 5
Siragusa S., De Angelis M., Calasso M., Campanella D., Minervini F., Di Cagno R., Gobbetti M., 2014. Fermentation and proteome profiles of Lactobacillus plantarum strains during growth under food-like conditions. Journal of proteomics, nr 96
Slonska A., Klimuszko D., 2010. Bakteriocyny probiotycznych paleczek z rodzaju Lactobacillus. Postepy Mikrobiologii, tom 10, nr 2
Smith J. L., Palumbo S. A., 1981. Microorganisms as food additives. Journal of Food Protection, nr 44
Staniewicz J., 2009. Jakosc mlecznych napojow fermentowanych suplementowanych dodatkiem pochodzenia roslinnego. Zeszyty Naukowe Akademii Morskiej w Gdyni, nr 5 Steinka I., 2011. Wybrane aspekty stosowania probiotykow. Annales Academiae Medicae Gedanensis, nr 41
Strus M., 1998. Nowa metoda oceny antagonistycznego dzialania bakterii kwasu mlekowego (LAB) na wybrane, chorobotworcze bakterie wskaznikowe. Medycyna Doswiadczalna i Mikrobiologia, nr 50
Slizewska K., Biernasiak J., Libudzisz Z., 2006. Probiotyki jako alternatywa dla antybiotykow. Zeszyty Naukowe Politechniki Lodzkiej. Chemia spozywcza i biotechnologia, z. 70
Trafalska E., Grzybowska K., 2004. Probiotyki - alternatywa dla antybiotykow? Wiadomosci lekarskie, tom 57, nr. 9-10
Trojanowska K., Giebel H., Golebiowska B., 2009. Mikrobiologia zywnosci. Wydawnictwo Uniwersytetu Przyrodniczego w Poznaniu
Trzaskowska M., 2013. Probiotyki w produktach pochodzenia roslinnego. Zywnosc. Nauka.
Technologia. Jakosc., tom 39
Yonezawa S., Xiao J. Z., Odamaki T., Ishida T., Miyaji K., Yamada A., Yaeshima T., Iwatsuki K., 2010. Improved growth of bifidobacteria by co- cultivation with Lactococcus lactis subspecies. lactis. Journal of Dairy Science, nr 93
Zareba D., Ziarno M., 2011. Alternatywne probiotyczne napoje warzywne i owocowe. Bromatologia i Chemia Toksykologiczna, tom 54, nr 2.

## Claims

1. A composition of a functional probiotic-herbal preparation in liquid form, **characterized in that** it comprises
- 70 - 90 % vol. of medium mineralized mineral water comprising chloride, bicarbonate, sodium, calcium, magnesium, potassium, fluoride, iodine, selenium, with a slightly alkaline pH;
- 3.2 - 13.7 % vol. liquid herbal extract from *Phyllanthus emblica* - water, 35 % alcohol, Drug Extract Ratio (DER): 1:15;
- 1.7 - 6 % vol. liquid herbal extract from *Glycyrrhiza glabra* - water, 32 % alcohol, DER: 1:25;
- 1.5 - 6 % vol. liquid herbal extract from *Stevia rebaudiana* - water, DER: 1:25;
- 0.7 % vol. of *Lactobacillus plantarum* PCM 493 strain (Polish Collection of Microorganisms) with a minimum content of 10 _{*} 10⁹ CFU/ml;
- 0.7 % vol. of *Bacillus coagulans* DSM 2383 strain (DSMZ - German Collection of Microorganism and Cells Cultures GmbH) with a minimum content of 2 _{*} 10⁹ CFU/ml;
- 0.5 % vol. of *Lactobacillus reuteri* DSM 17509 strain (DSMZ - German Collection of Microorganism and Cells Cultures GmbH) with a minimum content of 10 _{*} 10⁹ CFU/ml;
- 0.7 % vol. of *Bifidobacterium longum subs. infantis* DSM 20088 strain (DSMZ - German Collection of Microorganism and Cells Cultures GmbH) with a minimum content of 5 _{*} 10⁹ CFU/ml;
- 1 % vol. of *Lactobacillus rhamnosus* DSM 20245 strain (DSMZ - German Collection of Microorganism and Cells Cultures GmbH) with a minimum content of 10 _{*} 10⁹ CFU/ml;
- 0.7 % vol. of Lactobacillus casei DSM 20011 strain (DSMZ - German Collection of Microorganism and Cells Cultures GmbH) with a minimum content of 10 _{*} 10⁹ CFU/ml.

2. The composition of a functional probiotic-herbal preparation in liquid form according to claim 1, **characterized in that,** it comprises
- 70 - 90 % vol. of medium mineralized mineral water comprising chloride, bicarbonate, sodium, calcium, magnesium, potassium, fluoride, iodine, selenium, with pH of 7.4

3. The composition of a functional probiotic-herbal preparation in liquid form according to claims 1-2, **characterized in that** the medium mineralized mineral water is spring water extracted from a Permian deposit in Kolobrzeg, preferably Jantar mineral water.

4. The composition of a functional probiotic-herbal preparation in liquid form as defined in claims 1-3, for use as a medicament.

5. The composition of a functional probiotic-herbal preparation in liquid form as defined in claims 1-4 for use as a medicament or adjunct in the treatment, alleviation and/or prevention of entities and disease conditions selected from:
digestive disorders,diarrhea, obesity, cardiovascular disease, gastric or duodenal ulcer diseases, fatty liver and its consequences, hepatic encephalopathy, intestinal disorders of various origins, including specific and non-specific inflammatory and of autoimmune origin, type II diabetes, metabolic disorders and disease entities resulting from them, allergies, hormonal disorders, chronic fatigue syndrome, occupational burnout syndrome, depression and low mood states, digestive system disorders resulting from antibiotic therapy.

6. The composition for use according to claim 5, **characterized in that** it is used for treating, alleviating and/or preventing the onset of non-specific intestinal disorders, irritable bowel syndrome, occupational burnout and/or depressed mood states.

7. The composition of a functional probiotic-herb preparation in liquid form as defined in claims 1-3, for use as a liquid probiotic agent.

8. The composition of a functional probiotic-herbal preparation in liquid form as defined in claims 1-3, for use as a liquid symbiotic supplement.

9. The non-medical use of a composition of a functional probiotic-herbal preparation in liquid form as defined in claim 1-3 as a symbiotic, probiotic, dietary supplement and/or functional beverage for supplementing, stabilizing the microbiome and/or restoring the proper gastrointestinal microbiome in an individual.

## Patentansprüche

1. Zusammensetzung eines funktionellen probiotisch-pflanzlichen Präparats in flüssiger Form, **dadurch gekennzeichnet, dass** es Folgendes umfasst
- 70 - 90 Vol.-% mittelmineralisiertes Mineralwasser, das Chlorid, Bikarbonat, Natrium, Kalzium, Magnesium, Kalium, Fluorid, Jod, Selen enthält, mit einem leicht alkalischen pH-Wert;
- 3,2 - 13,7 Vol.-% flüssiger Kräuterextrakt aus *Phyllanthus emblica* - Wasser, 35 % Alkohol, Drug Extract Ratio (DER): 1:15;
- 1,7 - 6 Vol.-% flüssiger Kräuterextrakt aus *Glycyrrhiza glabra* - Wasser, 32 % Alkohol, DER: 1:25;
- 1,5 - 6 Vol.-% flüssiger Kräuterextrakt aus *Stevia rebaudiana* - Wasser, DER: 1:25;
- 0,7 Vol.-% des Stammes *Lactobacillus plantarum* PCM 493 (Polnische Sammlung von Mikroorganismen) mit einem Mindestgehalt von 10 _{*} 10⁹ CFU/ml;
- 0,7 Vol.-% des Stammes *Bacillus coagulans* DSM 2383 (DSMZ - Deutsche Sammlung von Mikroorganismen und Zellkulturen GmbH) mit einem Mindestgehalt von 2 - 10⁹ CFU/ml;
- 0,5 Vol.-% des Stammes *Lactobacillus reuteri* DSM 17509 (DSMZ - Deutsche Sammlung von Mikroorganismen und Zellkulturen GmbH) mit einem Mindestgehalt von 10 _{*} 10⁹ CFU/ml;
- 0,7 Vol.-% des Stammes *Bifidobacterium longum subs. infantis* DSM 20088 (DSMZ - Deutsche Sammlung von Mikroorganismen und Zellkulturen GmbH) mit einem Mindestgehalt von 5 - 10⁹ CFU/ml;
- 1 Vol.-% *Lactobacillus rhamnosus* DSM 20245 (DSMZ - Deutsche Sammlung von Mikroorganismen und Zellkulturen GmbH) mit einem Mindestgehalt von 10 - 10⁹ CFU/ml;
- 0,7 Vol.-% des Stammes Lactobacillus casei DSM 20011 (DSMZ - Deutsche Sammlung von Mikroorganismen und Zellkulturen GmbH) mit einem Mindestgehalt von 10 _{*} 10⁹ CFU/ml.

2. Zusammensetzung eines funktionellen probiotisch-pflanzlichen Präparats in flüssiger Form nach Anspruch 1, **dadurch gekennzeichnet, dass** es Folgendes umfasst
- 70 - 90 Vol.-% mittelmineralisiertes Mineralwasser, das Chlorid, Bikarbonat, Natrium, Kalzium, Magnesium, Kalium, Fluorid, Jod, Selen enthält, mit einem pH-Wert von 7,4.

3. Zusammensetzung eines funktionellen probiotisch-pflanzlichen Präparats in flüssiger Form nach einem der Ansprüche 1-2, **dadurch gekennzeichnet, dass** das mittelmineralisierte Mineralwasser ein Quellwasser ist, das aus einer permischen Lagerstätte in Ko 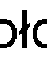 obrzeg (Kolberg) gewonnen wird, vorzugsweise ein Jantar-Mineralwasser.

4. Zusammensetzung eines funktionellen probiotisch-pflanzlichen Präparats in flüssiger Form gemäß den Ansprüchen 1-3 zur Verwendung als Arzneimittel.

5. Die Zusammensetzung eines funktionellen probiotisch-pflanzlichen Präparats in flüssiger Form, wie es in den Ansprüchen 1-4 definiert ist, zur Verwendung als Medikament oder Zusatz bei der Behandlung, Linderung und/oder Vorbeugung von Einheiten und Krankheitszuständen, ausgewählt aus:
Verdauungsstörungen, Durchfall, Fettleibigkeit, kardiovaskuläre Erkrankungen, Magen- oder Zwölffingerdarmgeschwüre, Fettleber und ihre Folgen, hepatische Enzephalopathie, Darmerkrankungen verschiedenen Ursprungs, einschließlich spezifischer und unspezifischer Entzündungen und Autoimmunerkrankungen, Typ-II-Diabetes, Stoffwechselstörungen und daraus resultierende Krankheiten, Allergien, hormonelle Störungen, chronisches Müdigkeitssyndrom, berufliches Burnout-Syndrom, Depressionen und Niedergeschlagenheit, Störungen des Verdauungssystems infolge einer Antibiotikatherapie.

6. Die Zusammensetzung zur Verwendung nach Anspruch 5, **dadurch gekennzeichnet, dass** sie zur Behandlung, Linderung und/oder Vorbeugung des Auftretens von unspezifischen Darmerkrankungen, Reizdarmsyndrom, beruflichem Burnout und/oder Depressionen verwendet wird.

7. Zusammensetzung eines funktionellen probiotisch-pflanzlichen Präparats in flüssiger Form gemäß den Ansprüchen 1-3 zur Verwendung als flüssiges probiotisches Mittel.

8. Zusammensetzung eines funktionellen probiotisch-pflanzlichen Präparats in flüssiger Form gemäß den Ansprüchen 1-3 zur Verwendung als flüssiges symbiotisches Ergänzungsmittel.

9. Die nicht-medizinische Verwendung einer Zusammensetzung eines funktionellen probiotisch-pflanzlichen Präparats in flüssiger Form, wie in den Ansprüchen 1 bis 3 definiert, als symbiotisches, probiotisches, diätetisches Ergänzungsmittel und/oder funktionelles Getränk zur Ergänzung, Stabilisierung des Mikrobioms und/oder Wiederherstellung des richtigen gastrointestinalen Mikrobioms in einem Individuum.

## Revendications

1. Composition d'une préparation fonctionnelle à base de probiotiques et d'herbes sous forme liquide, **caractérisée en ce qu'**elle comprend
- 70 - 90 % vol. d'eau minérale moyennement minéralisée comprenant du chlorure, du bicarbonate, du sodium, du calcium, du magnésium, du potassium, du fluor, de l'iode, du sélénium, avec un pH légèrement alcalin ;
- 3,2 - 13,7 % vol. d'extrait liquide d'herbe de *Phyllanthus emblica* - eau, 35 % d'alcool, le rapport plante/extrait (DER) : 1:15;
- 1,7 - 6 % vol. d'extrait liquide d'herbe de *Glycyrrhiza glabra* - eau, 32 % d'alcool, DER : 1:25 ;
- 1,5 - 6 % vol. d'extrait liquide d'herbe de *Stevia rebaudiana* - eau, DER : 1:25 ;
- 0,7 % vol. de la souche *Lactobacillus plantarum* PCM 493 (Collection polonaise de micro-organismes) avec une teneur minimale de 10 * 10⁹ CFU/ml ;
- 0,7 % vol. de la souche *Bacillus coagulans* DSM 2383 (DSMZ - Collection allemande de cultures de micro-organismes et de cellules, GmbH) avec une teneur minimale de 2 * 10⁹ CFU/ml ;
- 0,5 % vol. de la souche *Lactobacillus reuteri* DSM 17509 (DSMZ - Collection allemande de cultures de micro-organismes et de cellules, GmbH) avec une teneur minimale de 10 * 10⁹ CFU/ml ;
- 0,7 % vol. de la souche *Bifidobacterium longum subs. infantis* DSM 20088 (DSMZ - Collection allemande de cultures de micro-organismes et de cellules, GmbH) avec une teneur minimale de 5 _{*} 10⁹ CFU/ml ;
- 1 % vol. de la souche *Lactobacillus rhamnosus* DSM 20245 (DSMZ - Collection allemande de cultures de micro-organismes et de cellules, GmbH) avec une teneur minimale de 10 * 10⁹ CFU/ml ;
- 0,7 % vol. de la souche Lactobacillus casei DSM 20011 (DSMZ - Collection allemande de cultures de micro-organismes et de cellules, GmbH) avec une teneur minimale de 10 * 10⁹ CFU/ml ;

2. Composition d'une préparation fonctionnelle à base de probiotiques et d'herbes sous forme liquide selon la revendication 1, **caractérisée en ce qu'**elle comprend
- 70 - 90 % vol. d'eau minérale moyennement minéralisée comprenant du chlorure, du bicarbonate, du sodium, du calcium, du magnésium, du potassium, du fluor, de l'iode, du sélénium, avec un pH de 7,4.

3. Composition d'une préparation fonctionnelle à base de probiotiques et d'herbes sous forme liquide selon les revendications 1-2, **caractérisée en ce que** l'eau minérale moyennement minéralisée est de l'eau de source extraite d'un gisement permien à Koobrzeg, de préférence de l'eau minérale Jantar.

4. Composition d'une préparation fonctionnelle à base de probiotiques et d'herbes sous forme liquide telle que définie dans les revendications 1 à 3, pour une utilisation en tant que médicament.

5. Composition d'une préparation fonctionnelle à base de probiotiques et d'herbes sous forme liquide telle que définie dans les revendications 1 à 4 pour utilisation en tant que médicament ou adjuvant dans le traitement, l'atténuation et/ou la prévention d'entités et d'états pathologiques sélectionnés parmi :
troubles digestifs, diarrhée, obésité, maladies cardiovasculaires, ulcères gastriques ou duodénaux, stéatose hépatique et ses conséquences, encéphalopathie hépatique, troubles intestinaux d'origines diverses, y compris inflammatoires spécifiques et non spécifiques et d'origine auto-immune, diabète de type II, troubles métaboliques et entités pathologiques qui en résultent, allergies, troubles hormonaux, syndrome de fatigue chronique, syndrome d'épuisement professionnel, dépression et états de mauvaise humeur, troubles du système digestif résultant d'une antibiothérapie.

6. Composition pour utilisation selon la revendication 5, **caractérisée en ce qu'**elle est utilisée pour traiter, atténuer et/ou prévenir le déclenchement de troubles intestinaux non spécifiques, syndrome du côlon irritable, épuisement professionnel et/ou états d'humeur dépressive.

7. Composition d'une préparation fonctionnelle à base de probiotiques et d'herbes sous forme liquide telle que définie dans les revendications 1 à 3, pour une utilisation en tant qu'agent probiotique liquide.

8. Composition d'une préparation fonctionnelle à base de probiotiques et d'herbes sous forme liquide telle que définie dans les revendications 1 à 3, pour une utilisation en tant que supplément symbiotique liquide.

9. Utilisation non médicale d'une composition d'une préparation fonctionnelle à base de probiotiques et d'herbes sous forme liquide telle que définie dans les revendications 1 à 3 en tant que symbiotique, probiotique, supplément alimentaire et/ou boisson fonctionnelle pour supplémenter, stabiliser le microbiome et/ou restaurer le microbiome gastro-intestinal approprié chez un individu.
